**Europäisches Patentamt**

**European Patent Office**

**Office européen des brevets**

(19)

(11) Veröffentlichungsnummer : **0 071 879**
**B1**

(12) **EUROPÄISCHE PATENTSCHRIFT**

(45) Veröffentlichungstag der Patentschrift :
10.09.86

(21) Anmeldenummer : 82106812.9

(22) Anmeldetag : 28.07.82

(51) Int. Cl.⁴ : **A 61 K 7/00, A 61 K 9/06**

(54) **Cremegrundlage.**

(30) Priorität : 05.08.81 DE 3131006

(43) Veröffentlichungstag der Anmeldung :
16.02.83 Patentblatt 83/07

(45) Bekanntmachung des Hinweises auf die Patenterteilung : 10.09.86 Patentblatt 86/37

(84) Benannte Vertragsstaaten :
AT BE CH DE FR GB IT LI NL SE

(56) Entgegenhaltungen :
H. JANISTYN: "Handbuch der Kosmetika und Riechstoffe", 3. Auflage, Band 1, 1978, Seiten 436-439, Dr. Alfred Hüthig Verlag, Heidelberg, DE.

(73) Patentinhaber : **Henkel Kommanditgesellschaft auf Aktien**
**Postfach 1100 Henkelstrasse 67**
**D-4000 Düsseldorf-Holthausen (DE)**

(72) Erfinder : **Scheuffgen, Ingeborg**
**Spulgasse 3**
**D-4040 Neuss (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft eine neue, nicht selbst emulgierende Cremegrundlage auf der Basis von Cetylstearylalkohol, Glycerin-mono-distearat, Cetylpalmitat und $C_{12}$-$C_{18}$-Triglycerid.

Bisher wurden für die Herstellung von kosmetischen oder pharmazeutischen Emulsionen vorwiegend Fettalkohole, Partialglyceride oder Mischungen davon eingesetzt. All diese Konsistenzgeber sind nicht ganz befriedigend. Bei Emulsionen auf der Basis von Fettalkoholen tritt ein zu starker Viskositätsanstieg während der Lagerung auf und die Emulsionen haben meist eine inhomogene Struktur. Als nachteilig wird auch empfunden, daß sie beim Einreiben auf der Haut weisseln und daß sie einen unangenehmen Hauteindruck hinterlassen. Bei Emulsionen auf der Basis von Partialglyceriden wurde ein starker Viskositätabfall während der Lagerung bei Raumtemperatur und beim Herstellprozeß eine eintretende Gelphase festgestellt. Desweiteren werden relativ hohe Einsatzmengen, ca. 15 bis 16 Gew.-% bezogen auf die Gesamtzubereitung, benötigt. Um diese Nachteile zu vermeiden wurde versucht, Kombinationen von Fettalkoholen und Partialglyceriden einzusetzen. Aber auch diese Emulsionen zeigen keine voll befriedigenden Eigenschaften.

Aufgabe der vorliegenden Erfindung ist es daher, eine nicht selbst emulgierende Cremegrundlage bereitzustellen, welche die oben genannten Nachteile nicht aufweist.

Es ist gelungen, durch Kombination verschiedener bekannter Konsistenzgeber im ganz bestimmten Mengenverhältnis zueinander eine optimale Cremegrundlage bereitzustellen. Gegenstand der vorliegenden Erfindung ist eine Cremegrundlage bestehend aus

70 % Glycerin-mono-distearat
10 % Cetylstearylalkohol
10 % Cetylpalmitat
10 % $C_{12}$-$C_{18}$-Triglycerid

Es hat sich gezeigt, daß das Mengenverhältnis der einzelnen Komponenten zueinander von wesentlicher Bedeutung ist. Wie aus beiliegender Tabelle hervorgeht, tritt schon bei Weglassen nur einer Komponente oder bei geringfügiger Variation der Mengenverhältnisse eine wesentliche Produktverschlechterung ein.

(Siehe Tabelle Seite 3 f.)

| | I | II | III | IV | V | VI | VII | VIII | IX |
|---|---|---|---|---|---|---|---|---|---|
| Glycerin-mono-distearat | 60 | 60 | 70 | 65 | 40 | 60 | 70 | 60 | 70 |
| Cetylpalmitat | 10 | 15 | 10 | 20 | 20 | 20 | 15 | 15 | 10 |
| Cetylstearylalkohol | 20 | 15 | 20 | 15 | 20 | 20 | 15 | 10 | 10 |
| Triglycerid $C_{12}$-$C_{18}$ | 10 | 10 | – | – | 20 | – | – | 15 | 10 |
| Testrezepturen: | | | | | | | | | |
| Konsistenz | m.weich | weich | m.weich | weich | m.weich | m.weich | weich | weich | weich |
| Struktur | inhomogen | l.inhomogen | stark inhomogen | glatt | inhomogen | l.inhomogen | glatt | l.inhomogen | glatt |
| Stabilität | stabil | stabil | stabil | stabil | stabil | stabil | stabil | stabil | stabil |
| Viskosität: mPas | | | | | | | | | |
| nach 1 Tag | 19.500 | 400 | 11.400 | 14.900 | getrennt | 2.600 | 28.300 | 6.200 | 6.200 |
| nach 12 Wochen | 187.500 | 112.500 | 212.000 | 200.000 | | 250.000 | 68.750 | 11.200 | 8.700 |

IX = erfindungsgemäße Zusammensetzung

0 071 879

Die neue Zusammensetzung erfüllt alle Anforderungen an eine gute Cremegrundlage ; sie zeichnet sich insbesondere durch :

a) Gute Viskositätsstabilität über längere Zeit
b) Glatte Struktur, glänzende Oberfläche der Emulsionen
c) Gute Verteilbarkeit auf der Haut ohne Weisseleffekt
d) Gute Temperaturstabilität
e) Vorteile hinsichtlich Lagerhaltung und Herstellung durch einen Konsistenzgeber

aus. Die erfindungsgemäße Cremegrundlage läßt sich mit allen üblichen Emulgatoren kombinieren.

Folgende Emulgatoren wurden allein oder kombiniert mit der oben angegebenen Grundlage in Cremes und flüssigen Emulsionen geprüft :

Fettalkoholsulfat
Fettalkoholoxethylat
Partialglyceridoxethylat
Fettsäureoxethylat

Dabei zeigte sich, daß die genannten positiven Eigenschaften durch die Wahl des Emulgators nicht beeinflußt werden.

Zur näheren Erläuterung der Erfindung werden einige Beispiele für Rezepturen von Cremes mit der erfindungsgemäßen Cremegrundlage gegeben :

## Beispiel 1

Tagescreme

| | | |
|---|---|---|
| Glycerin-mono-distearat | | 14 % |
| Erfindungsgemäße Cremegrundlage | | 14 % |
| Cetylstearylalkohol mit ca. 12 Mol Ethylenoxid | 1,5 % | 1,5 % |
| Cetylstearylalkohol mit ca. 20 Mol Ethylenoxid | 1,5 % | 1,5 % |
| Di-n-Butyladipat | 6 % | 6 % |
| 2-Octyldecanol | 6 % | 6 % |
| Glycerin 86 %ig | 6 % | 6 % |
| Wasser | 65 % | 65 % |

## Beispiel 2

Nährcreme

| | | |
|---|---|---|
| Glycerin-mono-distearat | | 16 % |
| Erfindungsgemäße Cremegrundlage | | 16 % |
| Cetylstearylalkohol mit ca. 12 Mol Ethylenoxid | 1,5 % | 1,5 % |
| Cetylstearylalkohol mit ca. 20 Mol Ethylenoxid | 1,5 % | 1,5 % |
| Myritol® 318 (Fettsäureglycerinester) | 10 % | 10 % |
| Ölsäuredecylester | 10 % | 10 % |
| Glycerin 86 %ig | 5 % | 5 % |
| Wasser | 56 % | 56 % |

## Beispiel 3

Hautemulsion

| | | |
|---|---|---|
| Glycerin-mono-distearat | | 8 % |
| Erfindungsgemäße Cremegrundlage | | 8 % |
| Cetylstearylalkohol mit ca. 12 Mol Ethylenoxid | 3 % | 3 % |
| Cetiol® SN (Isononansäureester von gesättigten $C_{16}$-$C_{18}$ Fettalkoholen) | 10 % | 10 % |
| Glycerin 86 %ig | 5 % | 5 % |
| Wasser | 74 % | 74 % |

## Beispiel 4

Hautemulsion

| | | |
|---|---|---|
| Glycerin-mono-distearat | | 8 % |

4

| | | |
|---|---|---|
| Erfindungsgemäße Cremegrundlage | | 8 % |
| Cetylstearylalkohol mit ca. 12 Mol Ethylenoxid | 3 % | 3 % |
| 2-Octyldecanol | 4 % | 4 % |
| Myritol® 318 (Fettsäureglycerinester) | 8 % | 8 % |
| Glycerin 86 %ig | 5 % | 5 % |
| Wasser | 72 % | 72 % |

**Patentanspruch**

Cremegrundlage, dadurch gekennzeichnet, daß sie aus

70 Gew.-% Glycerin-mono-distearat
10 Gew.-% Cetylstearylalkohol
10 Gew.-% Cetylpalmitat
10 Gew.-% $C_{12}$-$C_{18}$-Triglycerid

besteht.

**Claim**

A cream base characterized in that it consists of

70 % by weight glycerol mono-distearate
10 % by weight cetylstearyl alcohol
10 % by weight cetyl palmitate
10 % by weight $C_{12}$-$C_{18}$ triglyceride.

**Revendication**

Base de crème, caractérisée en ce qu'elle consiste en

70 % en poids de mono-distéarate de glycérine
10 % en poids d'alcool cétylstéarylique
10 % en poids de palmitate de cétyle
10 % en poids de triglycéride en $C_{12}$-$C_{18}$.

5